# EUROPÄISCHE PATENTANMELDUNG

(11) **EP 0 992 791 A2**
(43) Veröffentlichungstag der Anmeldung: **12.04.2000**
(21) Anmeldenummer: 99106391.8
(22) Anmeldetag: 27.03.1999
(51) Int. Cl.: G01N 29/10

(54) **Verfahren und Vorrichtung zur Feuchtemessung an Mauerwerksmaterialien mit Signalanalyse**

(30) Priorität: 08.10.1998 DE 19846241
(71) Anmelder: Institut für angewandte Informatik im Bauwesen e.V., 23966 Wismar (DE)
(72) Erfinder: Fehlauer, Klaus-Uwe, Prof. Dr. rer. nat, 23966 Wismar (DE); Schwarz, Wolfgang, Dr.-Ing., 18233 Neubukow (DE)
(74) Vertreter: Schnick, Achim

(57) **Zusammenfassung**

Es wird ein Verfahren zur Feuchtemessung an Mauerwerksmaterialien mittels Signalanalyse, bei der ein Impuls in das Mauerwerk eingekoppelt, der Baustoff in Mikroschwingung versetzt und das Meßsignal mittels eines Schwingungsaufnehmers ausgekoppelt und anschließend einer Meßwertanalyse unterzogen wird sowie eine Vorrichtung zum Durchführen eines solchen Verfahrens mit einem Impulsgeber, einem Empfänger und einer Auswerteeinheit, indem das vom Material beeinflußte Signal vom Empfänger aufgezeichnet und nach erfolgter Digitalisierung einer Fouriertransformation unterzogen wird, beschrieben. Aus der Zeitfunktion sowie aus der durch Fouriertransformation entstandenen Frequenzfunktion werden in einer Vorverarbeitung schwingungstechnische Meßgrößen bestimmt, die zu aussagefähigen Merkmalen weiterverarbeitet werden, die in einem Klassifikator mit Hilfe einer zuvor aus einer repräsentativen Menge von Stichproben gleichen Materials ermittelten Referenzmerkmalen den Feuchtewerten zugeordnet werden. Als Merkmale werden die Energie im Spektrum, die Standardabweichung oder andere statistische Merkmale eingesetzt, die auf den schwingungstechnischen Meßgrößen beruhen. Desweiteren wird eine Vorrichtung zur Durchführung des Verfahrens mit einem Impulsgeber 2, einem Meßempfänger 4, einer Meßwerterfassung 5 und einer Auswerteeinheit 8, die aus dem Baugruppen Fourieranalysator 9, Vorverarbeitung/Merkmalrechner 10, Referenzmerkmalspeicher 11 und Klassifikator 12 besteht, beschrieben.

## Beschreibung

Die vorliegende Erfindung betrifft ein Verfahren zur Feuchtebestimmung an Mauerwerksmaterialien mit Signalanalyse, bei der ein Impuls in das Mauerwerk eingekoppelt und das Meßsignal mittels eines Schwingungsempfängers ausgekoppelt und anschließend einer Signalanalyse unterzogen wird sowie eine Vorrichtung zum Durchführen eines solchen Verfahrens mit einem Impulsgeber, einem Empfänger und einer Auswerteeinheit.

Die Diagnostik von feuchtebedingten Schäden an Bauwerken stellt auf Grund vieler unterschiedlich wirkender Einflußgrößen eine sehr komplexe Problematik dar. Eine Vielzahl von Versuchen zeigt, daß direkte Meßverfahren nicht in der Lage sind, den Feuchte- und Salzgehalt eines geschädigten Bauwerkes klar zu beschreiben. Aus diesem Grund wird heute noch die Wäge-Darr-Methode zur Feuchtebestimmung am Mauerwerk bevorzugt. Diese hat aber den entscheidenden Nachteil, daß Bohrproben an entsprechenden Meßachsen vom Gebäude gewonnen werden müssen, die dann im Labor durch Wiegen und Trocknen auf den Feuchtegehalt hin untersucht werden. Für Denkmale ist dieses höchst bedenklich, weil irreparable, ästhetische Zerstörungen der Gebäudeoberfläche, Veränderungen in der Statik und hohe Zeitaufwendungen bei der Gewinnung von Bohrproben und anschließenden chemischphysikalischen Analysen im Labor auftreten. Auch für Nachuntersuchungen, um den Sanierungserfolg nachzuweisen, scheidet dieses Verfahren aus. Neuere Verfahren wie z.B. Radar sind in der Anwendung so teuer, so daß sie auf wenige wertvolle Objekte beschränkt bleibt. Deswegen wird vielerorts nach neuen indirekten Meßverfahren unter Ausnutzung unterschiedlicher physikalischer Effekte gesucht. Ultraschall ist in vielen Bereichen der Technik ein anerkanntes Prüfverfahren, wobei vorrangig die Laufzeit ausgewertet wird (Tombers, J.: Untersuchungen zur Salzverteilung in verbauten Natursteinen - Beiträge zum Thema Salzverwitterung und zur Ultraschallmeßtechnik an Sandsteinen. Dissertation Universität Saarbrücken 1991; Peschel, G.J.: Geophysikalische Methoden zur Ermittlung der Feuchte poröser Gesteine und ihre Anwendungsmöglichkeiten für die Untersuchung der Feuchte von Bauteilen. Methodische Studie, BIG-M GmbH, Technologiezentrum Warnemünde 1993 (unveröffentlicht).

In DE 3507206 wird ein klassisches Ultraschallverfahren zur Bestimmung von verschiedenen Gesteinseigenschaften beschrieben. Bei diesem Verfahren wird an trockenen und feuchten Gesteinen gemessen, jedoch nicht die Feuchte im Gestein bestimmt.

In DE 4233958 A1 werden ein Ultraschallmeßverfahren und eine Vorrichtung beschrieben, welche moderne Methoden der Meßwertauswertung nutzen. Hier werden über einen speziellen Geber Longitudinalwellen mit unterschiedlicher Ausrichtung genutzt, um den Gefügezustand von Gesteinen zu ermitteln. Die unterschiedlichen Ausbreitungsgeschwindigkeiten der axialen und radialen Wellenanteile werden über die Fouriertransformation ausgewertet. Die beiden Komponenten der Longitudinalwelle werden in der Auswertung herausgefiltert und getrennt ausgewertet. Um diesen Effekt zu verstärken, wurde ein spezieller Kopf entwickelt, der die axialen und radialen Wellenanteile selektiv abgibt. Eine Feuchtemessung ist mit dem genannten Verfahren nicht möglich.

Bekannt ist ferner durch die DE 4117091 A1 ein Verfahren zur Erstellung eines Klassifikationssystems zur Erkennung der Beschaffenheit eines Fahrbahnbelages. Bei diesem Verfahren wird ausschließlich im Zeitbereich gearbeitet. Hier werden Methoden der Objekterkennung ansatzweise genutzt, wobei ausschließlich die Varianz sowie Normierungen davon zur Anwendung kommen. Aus Messungen unter verschiedenen Bedingungen werden Varianzen als Merkmalvektor abgeleitet, wobei jede Messung einem Merkmal zugeordnet werden kann. Dieses System ist nur dazu geeignet, die Art eines Fahrbahnbelages aus dem Reflexionsvermögen von Ultraschallwellen zu bestimmen.

Die US 53633850 stellt ein Verfahren zur Untersuchung von Blut vor, bei dem ebenfalls die Objekterkennung mit einem Klassifikator zum Einsatz kommt.

Prinzipiell gilt ein nachhaltiger Unterschied zwischen der Bestimmung von Gesteinsarten bzw. deren Eigenschaften und der Bestimmung der Feuchte in kapillarporösen Baustoffen. Erst bei kapillarporösen Baustoffen tritt das Feuchteproblem auf, indem Feuchte aus dem Boden im Baustoff hochgezogen wird und sich im Kapillarraum anreichert. Deswegen sind die vorgenannten Verfahren zur Feuchtebestimmung nicht einsetzbar. Bei der Feuchtemessung an Mauerwerk wird die eigentliche Meßgröße von vielen Einflußgrößen überdeckt, so daß es nur unter Nutzung moderner Methoden der Informations- und Meßwertverarbeitung und der Objekterkennung möglich ist, die Nutzgrößen zu ermitteln.

Der Erfindung liegt daher die Aufgabe zugrunde, in einem Verfahren die empfangenen Meßsignale in Abhängigkeit von der Feuchte auswertbar zu machen und die Vorrichtung zur Durchführung des Verfahrens so zu gestalten, daß leistungsfähige Messungen ermöglicht werden.

Erfindungsgemäß wird die Aufgabe durch die Merkmale der Ansprüche 1 und 6 gelöst. Vorteilhafte Ausgestaltungen ergeben sich aus den Unteransprüchen.

Nach dem neuen Verfahren werden die sich durch die Feuchteeinlagerungen ändernden schwingungstechnischen Eigenschaften des kapillarporösen Baustoffs erfaßt und einer Auswertung zugänglich gemacht. Dazu wird von einem Geber ein Diracimpuls ausgesendet, der den Baustoff in Mikroschwingungen versetzt, diese werden von einem Schwingungsaufnehmer in einer Abtastung (Scan) aufgezeichnet und nach einer erfolgten Digitalisierung einer Fouriertransformation unterzogen, als Zwischengrößen werden mehrere schwingungstypische Charakteristika im Zeit- und Frequenzbereich abgeleitet, woraus dann aussagefähige Merkmale auf statistischer Grundlage berechnet werden, die in einem Klassifikator mit Hilfe einer zuvor aus einer repräsentativen Menge von Stichproben gleichen Materials ermittelten Vergleichsmerkmale den Feuchtewerten zugeordnet werden, als Merkmale die Energie im Spektrum, die Standardabweichung oder andere statistische Merkmale eingesetzt werden, die auf den schwingungstypischen Charakteristikas beruhen.

Vorrichtungsmäßig erfolgt die Lösung der Aufgabe dadurch, daß der Geber einen vom Impulsgenerator ausgelösten Impuls in das Mauerwerksmaterial einkoppelt, dieses vom Material veränderte Signal einem Schwingungsaufnehmer zugeführt wird, von der Meßwerterfassungseinheit, bestehend aus Analog/Digitalwandler, Fourieranalysator und Speicher, und der Auswerteeinheit, bestehend aus Schwingungsanalysator, Merkmalrechner, Referenzmerkmalspeicher und Klassifikator, zugeführt wird.

Jedes kapillarporöse System, wozu auch alle gängigen Mauerwerksmaterialien gehören, stellt ein akustisches Filter dar, dessen akustische Parameter Grenzfrequenz, Eigenfrequenz, Dämpfung, Güte, Signalleistung und Schwingungsverhalten (Einschwingzeit) von den jeweiligen Porenzwickeln abhängig sind. Feuchteeinlagerungen im freien Porenraum verändern diese Parameter. Bei der theoretischen Betrachtung wird das kapillarporöse System des Baustoffs als Reihen-/Parallelschwingkreis angesehen. Wird ein kurzer Nadelimpuls (Diracimpuls) über einen Ultraschallimpulsgeber in das Mauerwerksmaterial eingekoppelt, so wird das Porengefüge zum Schwingen angeregt. Je nach Feuchtegehalt im Porenraum ändert sich das Schwingungsverhalten. Die akustischen Grundparameter sind jeweils für eine Materialart, aus dem der Porenraum gebildet wird, typisch. Das Signalgemisch als Systemantwort kann über einen Meßwertaufnehmer aufgezeichnet werden. Über eine Fast-Fouriertransformation läßt sich das mittels AD-Wandler digitalisierte Signal vom Meßwertaufnehmer analysieren. In der Vorverarbeitung werden zunächst die schwingungstechnischen Eigenschaften Grenzfrequenz, Eigenfrequenz, Dämpfung, Güte, Signalleistung und Schwingungsverhalten (Einschwingzeit) ermittelt. Kapillarporöse Materialien sind in der Regel nicht sehr homogen, d.h., bei der Herstellung treten in der Zusammensetzung und im Gefüge größere Schwankungen auf. Dieses wirkt sich voll im Signalgemisch der Messung aus, so daß die unterschiedlichen Einflüsse aus Materialzusammensetzung, Gefügeschwankungen und Feuchteeinflüssen, bezogen auf die zu messende Feuchte, unscharf werden. Aus diesem Grunde werden verfahrensbedingt in Anlehnung an die Objekterkennung aus einer Abtastung (Scan) in der Vorverarbeitung mehrere schwingungstypische Systemkenngrößen vom kapillarporösen Baustoff ermittelt, die dann zu Merkmalen weiterverarbeitet werden.

Vorzugsweise wird dabei auf statistische Merkmale wie Energie unter dem Spektrum und Standardabweichung im Spektrum zurückgegriffen. Weitere statistische sowie auch deterministische Merkmale sind möglich, erhöhen aber den Aufwand. Die Mindestanzahl an verwendeten Merkmalen, um in Klassifikator eine klare Zuweisung zu erhalten, sollten zwei nicht unterschreiten. Die für den Klassifikator notwendigen Vergleichswerte sind von bekannten Materialproben Referenzmerkmale, die als Funktion der Feuchte abgespeichert im Referenzmerkmalspeicher abgelegt werden, zu ermitteln. Da auch hier statistische Einflüsse eine große Rolle spielen, sind diese sinnvollerweise aus einer Stichprobe zu ermitteln. Im Klassifikator erfolgt mit Hilfe von zuvor aus einer repräsentativen Menge von Stichproben gleichen Materials ermittelten Vergleichsmerkmalen die Zuordnung zu den Feuchtewerten. Vorzugsweise wird der Nächste-Nachbar-Klassifikator angewendet. Andere Klassifikatoren können auch eingesetzt werden.

Fehlzuweisungen sind nicht auszuschließen, da das Meßobjekt selbst in seiner inneren Struktur starken statistischen Schwankungen unterliegt und als zweiter Unsicherheitsfaktor die Ankopplungsprobleme der Meßgeber/-empfänger hinzukommen, die auch statistischen Schwankungen von nicht unerheblichen Ausmaß unterliegen. Um dieses zu vermeiden, wird eine Toleranzprüfung durchgeführt, indem einmal geprüft wird, ob die aktuelle Messung in den Toleranzschläuchen der abgespeicherten Referenzmerkmale liegt und die zweite Prüfung erfolgt, ob die Merkmale in der Zuweisung zu den Feuchtewerten innerhalb einer vorgegebenen Toleranz das gleiche Ergebnis liefern. Ist dieses nicht der Fall, wird die Messung zurückgewiesen.

Im folgenden soll die Erfindung anhand eines Ausführungsbeispieles näher erläutert werden. In der zugehörigen Zeichnung zeigen:
- Fig. 1: den Aufbau der erfindungsgemäßen Vorrichtung,

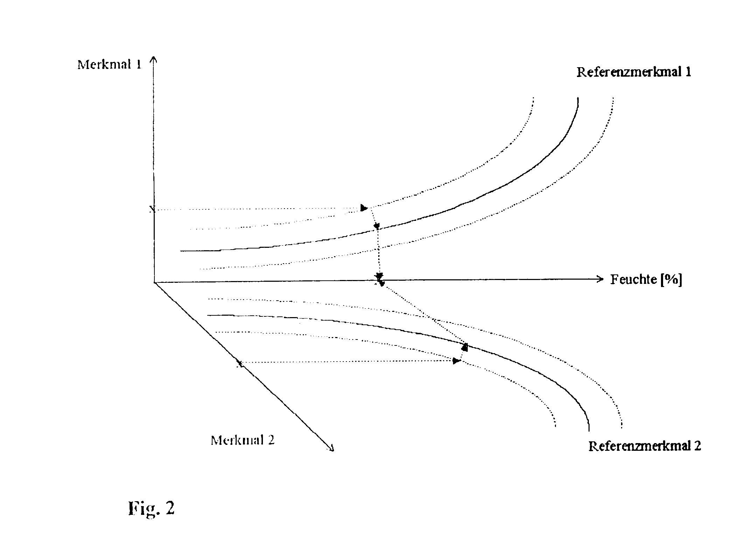

- Fig. 2: den Vorgang der Zuordnung zwischen Meßwert und Feuchtewert mit Hilfe der Referenzmerkmale.

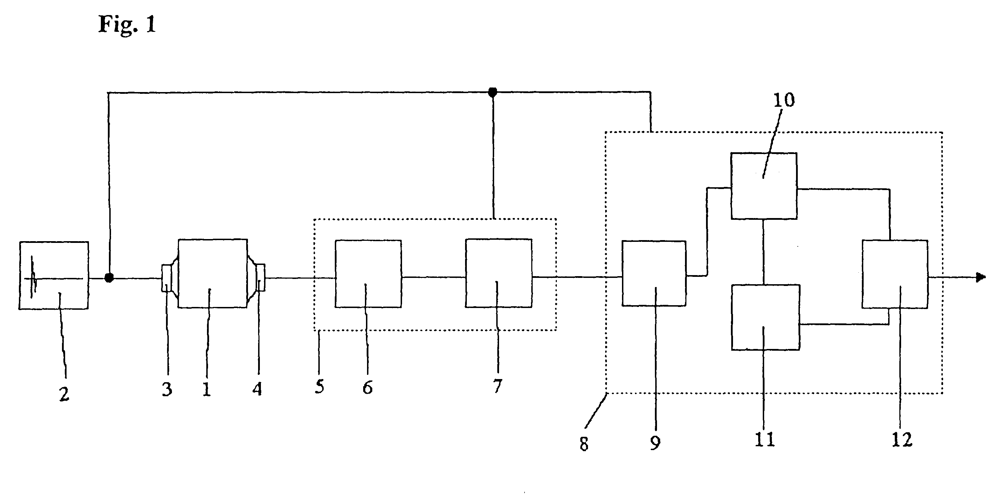

Die Vorrichtung zur Durchführung des Verfahrens ist in Fig. 1 dargestellt. Das Meßobjekt 1 ist mit einem Meßgeber 3 und einem Meßempfänger 4 gekoppelt. Der Meßgeber 3 wird von einem Impulsgenerator 2 angesteuert. Der Meßempfänger 4 liefert die Meßsignale an eine Meßwerterfassungseinheit 5, die aus einem A/D-Wandler 6 und einem Meßwertspeicher 7 besteht. Die verfahrensgemäße Auswertung der Meßwerte erfolgt in der Auswerteeinheit 8, die aus einem Fourieranalysator 9 für die schnelle Fouriertransformation, dem Merkmalrechner 10, dem Referenzmerkmalspeicher 11, in dem die zuvor aus einer Stichprobe ermittelten Referenzmerkmale abgelegt werden, und dem Klassifikator 12 besteht. Als Impulsgeneratorkann ein Ultraschallimpulsgenerator eingesetzt werden.

## Patentansprüche

1. Verfahren zur Feuchtebestimmung an Mauerwerksmaterialien mit Signalanalyse, bei der das Meßsignal mittels eines Schwingungsaufnehmers am Baustoff ausgekoppelt wird, die Meßwerte digitalisiert, in einer schnellen Fouriertransformation in den Frequenzbereich transformiert und gespeichert einer Auswerteeinheit zugeführt werden, dadurch gekennzeichnet, daß mittels eines Impulsstoßes der Baustoff in Mikroschwingungen versetzt wird, aus dem Zeitsignal und/oder Frequenzsignal einer Abtastung innerhalb einer Vorverarbeitung schwingungstechnische Eigenschaften des Baustoffes ermittelt werden, diese dann durch statistische Wichtung zu mehreren Merkmalen weiterverarbeitet werden und über einen Klassifikator, dessen Vergleichswerte aus zuvor ermittelten Referenzmerkmalen, die aus einer repräsentativen Menge des Prüfmaterials ermittelt wurden, eine Zuordnung zu einem Wert in der Feuchtetabelle erfolgt.

2. Verfahren nach Anspruch 1, dadurch gekennzeichnet, daß als Merkmale die Energie unter dem Spektrum und die Standardabweichung des Spektrums eingesetzt werden.

3. Verfahren nach Anspruch 1, dadurch gekennzeichnet, daß mindestens zwei oder mehr Merkmale ermittelt aus der Zeitfunktion oder Frequenzfunktion für die Bewertung herangezogen wird.

4. Verfahren nach Anspruch 1 und 3, dadurch gekennzeichnet, daß über den Nächster-Nachbar-Klassifikator die Zuweisung zur Feuchtetabelle erfolgt.

5. Verfahren nach Anspruch 1 bis 4, dadurch gekennzeichnet, daß die Referenzmerkmale in einem Selbstlernprozeß aus einer repräsentativen Menge von Mauerwerksproben ermittelt werden.

6. Vorrichtung zur Durchführung des Verfahrens nach Anspruch 1 mit einem Impulsgenerator, einem Meßgeber und einem Meßempfänger, dadurch gekennzeichnet, daß das vom Meßempfänger (4) aufgezeichnete Meßsignal der Meßwerterfassungseinheit (5), bestehend aus Analog/Digitalwandler (6) und Meßwertspeicher (7) und der Auswerteeinheit (8), bestehend aus Fourieranalysator (9), dem Merkmalrechner (10), dem Referenzmerkmalspeicher (11) und Klassifikator (12) zugeführt wird.

7. Vorrichtung nach Anspruch 6, dadurch gekennzeichnet, daß als Impulsgenerator ein Ultraschallimpulsgenerator (2) eingesetzt wird.

8. Vorrichtung nach Anspruch 6, dadurch gekennzeichnet, daß der ausgelöste Ultraschallimpuls zur Triggerung der Meßwerterfassungseinheit (5) und der Auswerteeinheit (8) verwendet wird.

9. Vorrichtung nach Anspruch 6, dadurch gekennzeichnet, daß Meßwerterfassungseinheit (5) und Auswerteeinheit (8) in off-line betrieben werden.
